# EUROPEAN PATENT APPLICATION

(11) **EP 4 714 363 A1**
(43) Date of publication of application: **25.03.2026**
(21) Application number: 24825516.8
(22) Date of filing: 12.03.2024
(51) Int. Cl.: A61B 8/06

(54) **ULTRASONIC DIAGNOSTIC DEVICE, MEDICAL INFORMATION PROCESSING DEVICE, INFORMATION PROCESSING METHOD, AND PROGRAM**

(30) Priority: 20.06.2023 JP 2023100608
(71) Applicant: Canon Kabushiki Kaisha, Tokyo 146-8501 (JP)
(72) Inventor: SASAKI, Shoya, Tokyo 146-8501 (JP); IIZUKA, Naoya, Tokyo 146-8501 (JP); SATO, Takeshi, Ohtawara-shi, Tochigi 324-8550 (JP); TAKAHASHI, Hiroki, Ohtawara-shi, Tochigi 324-8550 (JP)
(74) Representative: WESER & Kollegen Patentanwälte PartmbB
(86) International application number: PCT/JP2024/009505
(87) International publication number: WO 2024/262105

(57) **Abstract**

The present disclosure includes an ultrasonic diagnostic device including a first acquisition unit configured to acquire, based on an ultrasonic signal reflected in a living body, measurement data including tissue-derived information and blood-flow-derived information, a second acquisition unit configured to acquire, based on blood flow data obtained by extracting or emphasizing the blood-flow-derived information of the measurement data, a deformation amount between a plurality of frames, and a generation unit configured to generate display image data by aligning feature points in the blood-flow-derived information between the frames based on the deformation amount and then synthesizing the aligned feature points for the plurality of frames.

## Description

### [Technical Field]

The present invention relates to an ultrasonic diagnostic device, a medical information processing device, an information processing method, and a program.

### [Background Art]

An ultrasonic diagnostic device is widely used to observe and diagnose a blood flow in a living body. The ultrasonic diagnostic device generates and displays blood flow information from a reflected wave of an ultrasonic wave by a Doppler method based on the Doppler effect. Examples of the blood flow information generated and displayed by the ultrasonic diagnostic device include a color Doppler image, a Doppler waveform (Doppler spectrum), and the like.

The color Doppler image is captured by a color flow mapping (CFM) method. In the CFM method, ultrasonic waves are transmitted and received a plurality of times on a plurality of scanning lines. Then, by applying a moving target indicator (MTI) filter to a data string at the same position, a signal (clutter signal) derived from a stationary tissue or a slow-moving tissue is suppressed, and a signal derived from a blood flow is extracted. In the CFM method, pieces of blood flow information such as the velocity of a blood flow, the dispersion of a blood flow, and the power of a blood flow are estimated from this blood flow signal, and the distribution of an estimation result is displayed as a Doppler image.

In a B-mode image and a Doppler image, it is known that resolution deteriorates by a point spread function (PSF) determined by a wavelength of a transmission ultrasonic wave, a transmission/reception opening width, and the like. There is a solution such as increasing the frequency of the transmission ultrasonic wave, but there is also a limit to a frequency band of a probe, so that the resolution of an image that can be acquired is limited.

Non Patent Literature 1 describes a super resolution technique for a blood flow image that achieves a resolution of about 115 of a transmission ultrasonic wavelength. By extracting and integrating portions having high pixel values from a large number of blood flow images obtained in time series, a blood flow image having improved resolution is generated.

### [Citation List]

### [Non Patent Literature]

[Non Patent Literature 1] Jorgen Arendt Jensen et al., "Fast super resolution ultrasound imaging using the erythrocytes," Proc. SPIE 12038, Medical Imaging 2022: Ultrasonic Imaging and Tomography, 120380E (4 April 2022)

### [Summary of Invention]

### [Technical Problem]

In Non Patent Literature 1, first, tissue motion estimation in an ultrasonic image is performed by speckle tracking, and based on the estimation result, ultrasonic images of all frames are aligned with an image of a reference frame. Then, after a blood flow image is obtained by removing a tissue component from the aligned ultrasonic image, a portion having a high pixel value in the blood flow image of each frame is extracted and integrated.

However, for example, in an ultrasonic image in which few characteristic structures are included in the tissue or an ultrasonic image in which speckles are dominant, estimation accuracy of speckle tracking deteriorates, and deviation of the blood vessel position may not be sufficiently corrected. In such a case, the method of Non Patent Literature 1 cannot obtain high-resolution blood flow information.

The present invention has been made in view of the above-described problems, and an object thereof is to provide a technique for obtaining high-resolution blood flow information.

### [Solution to Problem]

The present disclosure includes an ultrasonic diagnostic device including a first acquisition unit configured to acquire, based on an ultrasonic signal reflected in a living body, measurement data including tissue-derived information and blood-flow-derived information, a second acquisition unit configured to acquire, based on blood flow data obtained by extracting or emphasizing the blood-flow-derived information of the measurement data, a deformation amount between a plurality of frames, and a generation unit configured to generate display image data by aligning feature points in the blood-flow-derived information between the frames based on the deformation amount and then synthesizing the aligned feature points for the plurality of frames.

### [Advantageous Effects of Invention]

According to the present invention, high-resolution blood flow information can be obtained.

### [Brief Description of Drawings]

[Fig. 1]

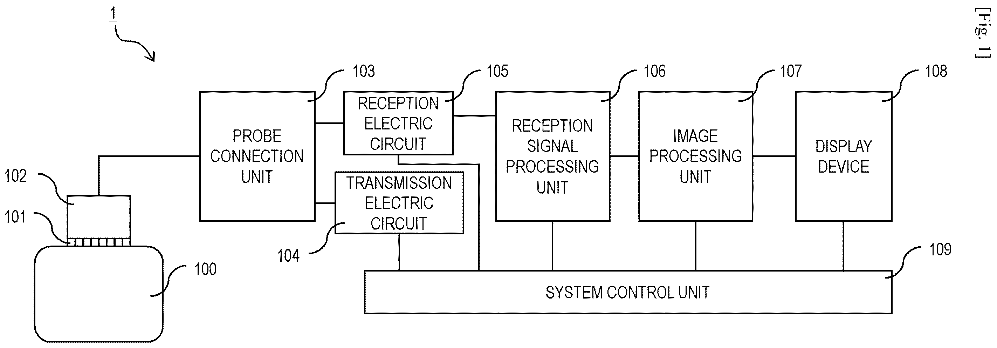

   Fig. 1 is a block diagram illustrating an example of a configuration of an ultrasonic diagnostic device.
[Fig. 2]

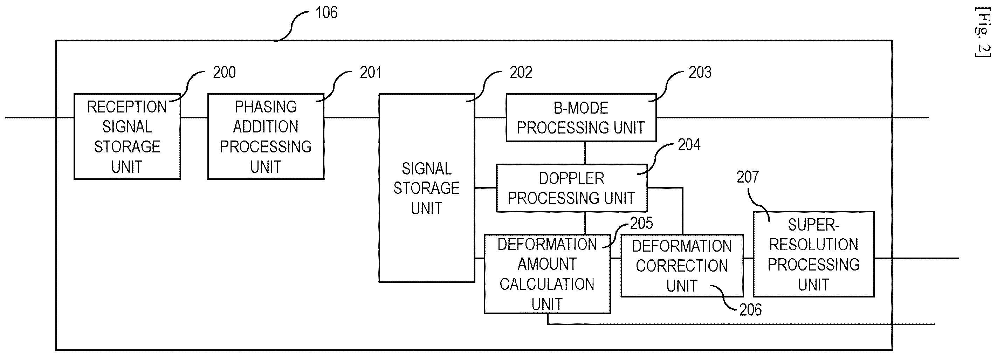

   Fig. 2 is a block diagram illustrating an example of a function of a reception signal processing block.
[Fig. 3]

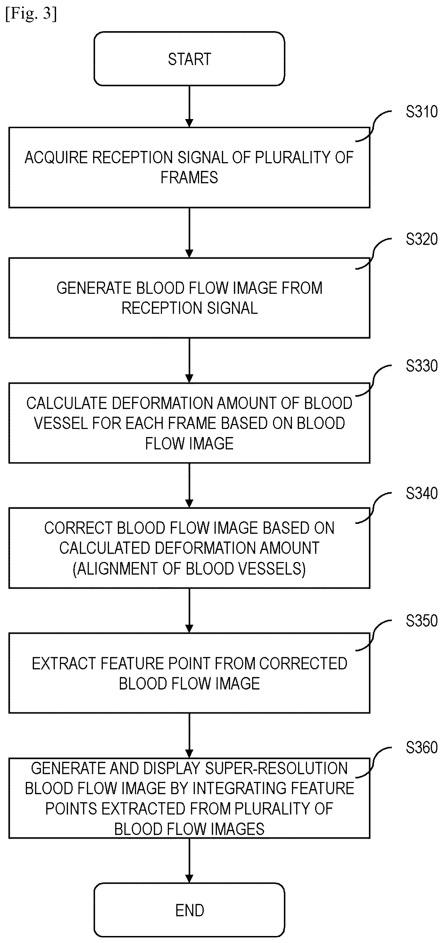

   Fig. 3 is a diagram illustrating a flow of super-resolution image generation processing according to a first embodiment.
[Fig. 4]

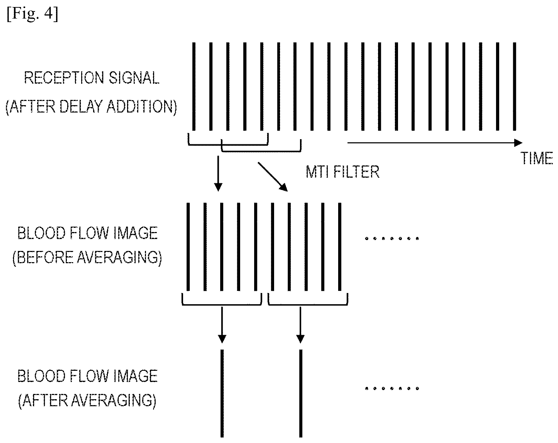

   Fig. 4 is a diagram for description of setting of a frame of blood flow image generation according to the first embodiment.
[Fig. 5]

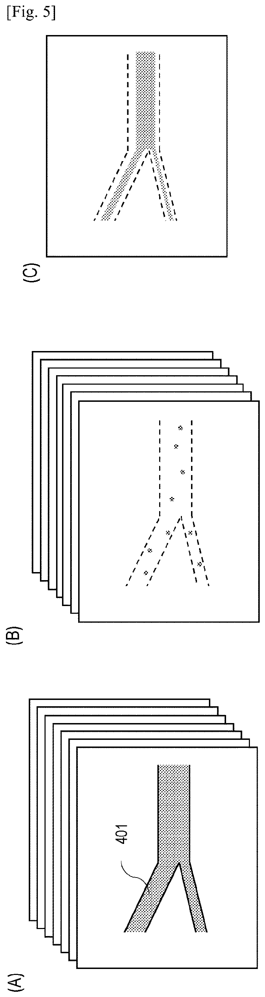

   Figs. 5A to 5C are diagrams for description of generation of a super-resolution image according to the first embodiment.
[Fig. 6]

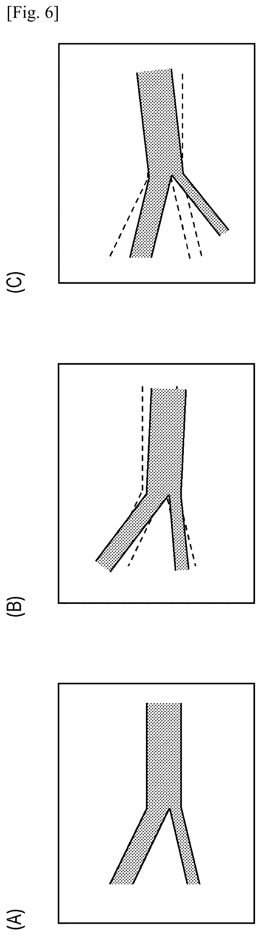

   Figs. 6A to 6C are diagrams for description of correction of a deformation amount accompanied by non-linear deformation in a blood flow image.
[Fig. 7]

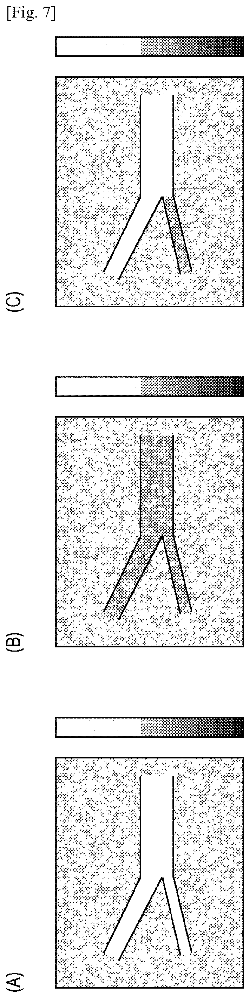

   Figs. 7A to 7C are diagrams for description of selection of a reference frame when a displacement amount is calculated.
[Fig. 8]

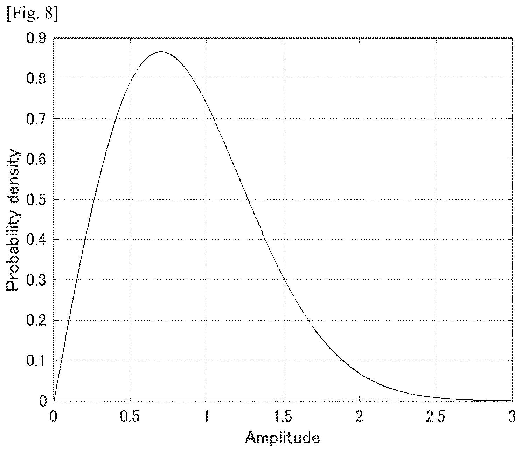

   Fig. 8 is a diagram for description of the Rayleigh distribution.
[Fig. 9]

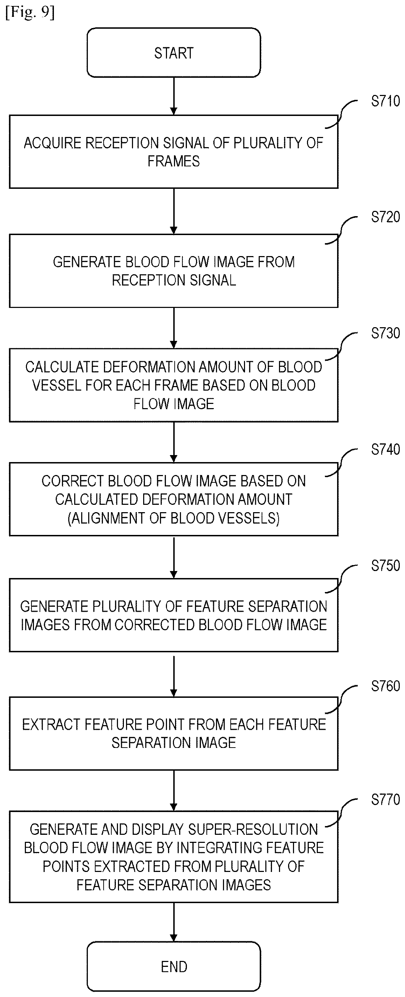

   Fig. 9 is a diagram illustrating a flow of super-resolution image generation processing according to a second embodiment.
[Fig. 10]

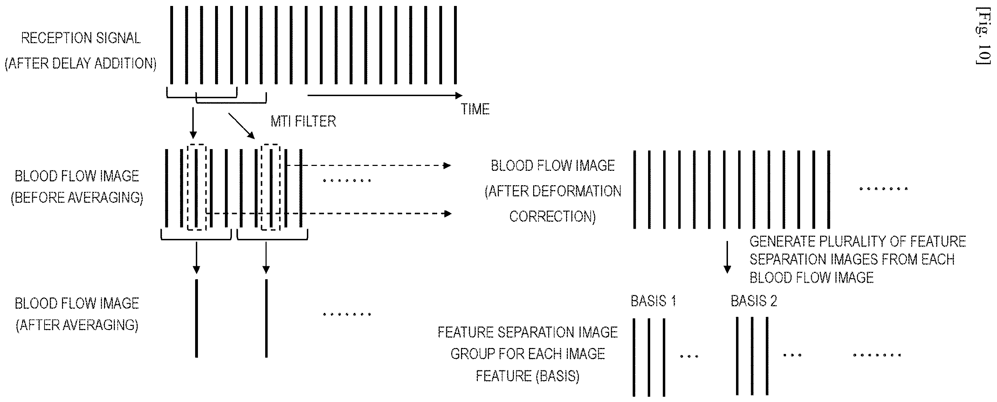

   Fig. 10 is a diagram for description of setting of a frame of blood flow image generation according to the second embodiment.
[Fig. 11]

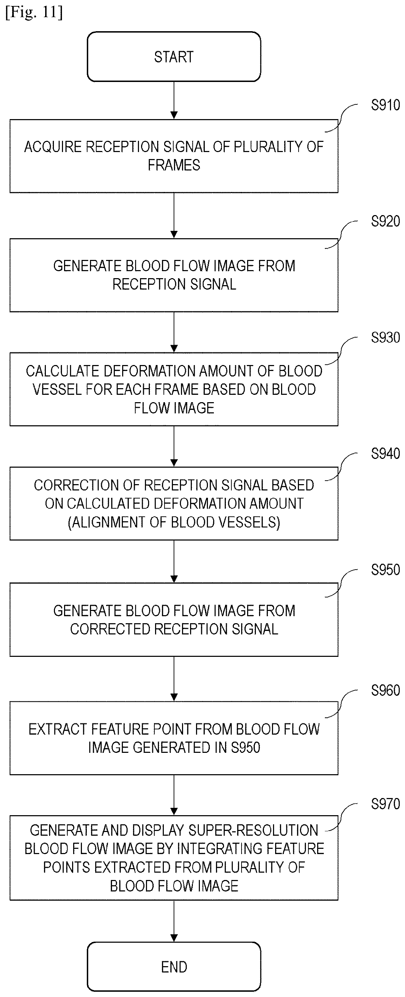

   Fig. 11 is a diagram illustrating a flow of super-resolution image generation processing according to a third embodiment.
[Fig. 12]

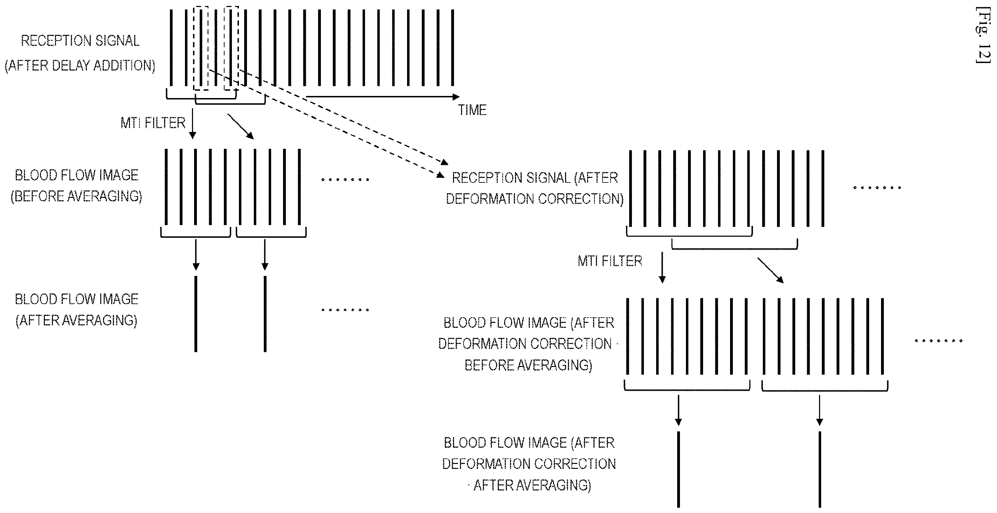

   Fig. 12 is a diagram for description of setting of a frame of blood flow image generation according to the third embodiment.
[Fig. 13]

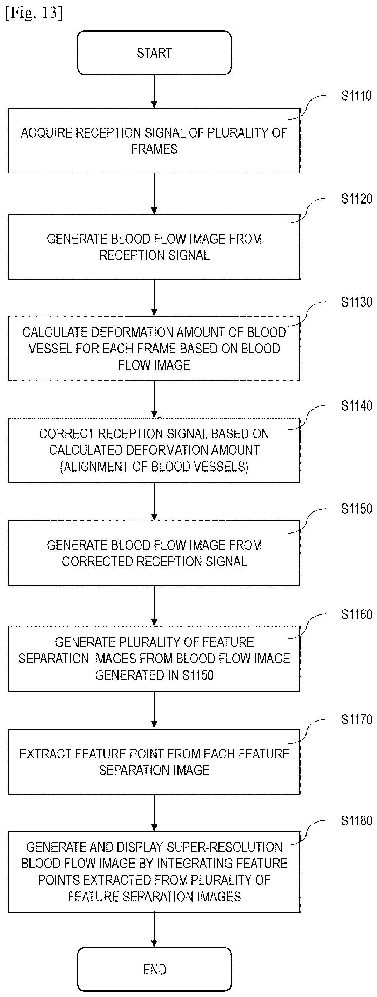

   Fig. 13 is a diagram illustrating a flow of super-resolution image generation processing according to a fourth embodiment.
[Fig. 14]

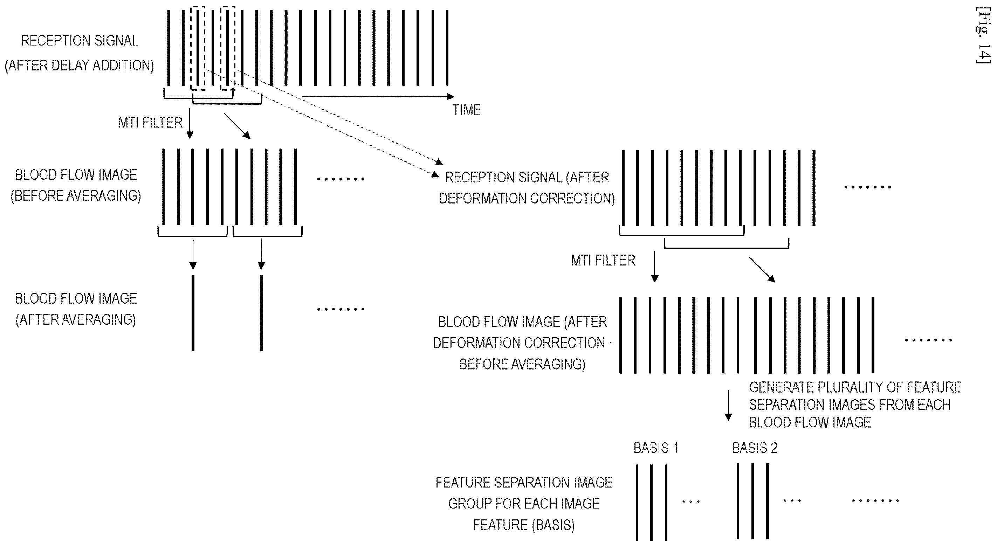

   Fig. 14 is a diagram for description of setting of a frame of blood flow image generation according to the fourth embodiment.

### [Description of Embodiments]

As described above, in the conventional super-resolution technique, displacement of the blood vessel position between frames is corrected by performing alignment between ultrasonic images by speckle tracking. The ultrasonic image includes tissue-derived information (hereinafter also simply referred to as a "tissue component") and blood-flow-derived information (hereinafter also simply referred to as a "blood flow component"). In speckle tracking, attention is paid to similarity of speckles appearing in these tissue components, and speckle patterns are compared and tracked between frames, thereby estimating correspondence and displacement of tissues between the frames. In this method, for example, in a case where a characteristic structure is shown in an ultrasonic image, such as a myocardium in an echocardiogram, a certain level of estimation accuracy can be expected. However, as described above, the estimation accuracy deteriorates in an ultrasonic image including few characteristic structures or an ultrasonic image in which speckles are dominant.

Therefore, the present inventors have obtained an idea of a new approach of performing alignment of a blood vessel using an image feature of a blood flow component instead of performing alignment of a blood vessel using an image feature of a tissue component in an ultrasonic image. By using the image feature of the blood vessel (blood flow) itself to be aligned, it can be expected that higher accuracy can be obtained than in the conventional method (method using the image feature of the tissue component). However, in a general ultrasonic image, image features of tissue components appear overwhelmingly more than blood flow components. Therefore, even if the present approach is simply applied to an ultrasonic image, it is difficult to accurately capture a deformation amount of a blood vessel region for each frame.

In order to solve such a technical problem, the following procedure is adopted in the ultrasonic diagnostic device according to the present embodiment.

First, a first acquisition unit acquires, based on an ultrasonic signal reflected in a living body, measurement data including tissue-derived information (tissue component) and blood-flow-derived information (blood flow component). The "measurement data" may be, for example, a reception signal obtained by transmitting and receiving an ultrasonic signal, data obtained by performing phasing addition and orthogonal detection processing on the reception signal, and the like. Note that the blood-flow-derived information may include not only information derived from blood but also information derived from a contrast medium in blood. Time series measurement data for a plurality of frames is acquired at a predetermined frame rate.

Next, a second acquisition unit acquires a deformation amount between a plurality of frames based on blood flow data in which the blood-flow-derived information of the measurement data is extracted or emphasized. The "extracting blood-flow-derived information" is, for example, an operation of selectively extracting an image feature of a blood flow component from measurement data. The "emphasizing blood-flow-derived information" is, for example, an operation of making an image feature of a blood flow component relatively more prominent than an image feature of a tissue component. Note that the second acquisition unit may obtain blood flow data by performing processing of extracting or emphasizing the blood-flow-derived information, or conversely, may obtain blood flow data by performing processing of removing or reducing tissue-derived information. For example, the processing of the second acquisition unit may include processing of calculating information (also referred to as "blood flow information") such as a velocity, a dispersion value, and a power value of a blood flow by a Doppler method. The "deformation amount" is an index representing relative displacement or deformation with respect to a reference position or shape. The second acquisition unit may acquire the deformation amount based on a blood vessel region (for example, the position or shape of a vessel region) in the blood flow data. A method of obtaining a deformation amount and a method of holding a parameter are arbitrary, and may be appropriately designed according to an algorithm of correction processing (processing for aligning positions of blood vessel regions between frames) in the subsequent stage.

Next, a generation unit generates display image data by aligning feature points in the blood-flow-derived information based on a deformation amount and then synthesizing the feature points for a plurality of frames. The "blood-flow-derived information" may be extracted from blood flow data acquired by the second acquisition unit, or may be extracted from measurement data or data generated from the blood flow data. The "feature point" may be a portion (that is, a portion having high probability of indicating a presence location of a blood flow) in which a feature of a blood flow is particularly strongly expressed in the blood-flow-derived information. For example, when a feature point is extracted from an image having blood-flow-derived information (a velocity, a dispersion value, and a power value of a blood flow) as a pixel value, a pixel having a maximum pixel value in the image, a pixel having a pixel value equal to or greater than a predetermined threshold value, or the like may be extracted as the feature point. A range (extraction range) of the pixel value from which the feature point is extracted can be arbitrarily set. "Synthesis" is an operation of combining information of a plurality of feature points extracted from each of the plurality of frames to generate one blood flow image, and can be processing of, for example, integrating a plurality of feature points on one image (accumulation; plotting). The "display image data" is data obtained by imaging high-resolution blood flow information displayed as a processing result, and is also referred to as an ultra-high-resolution blood flow image.

According to the above-described procedure, the deformation amount is obtained based on the blood flow data in which the blood-flow-derived information is dominant, whereby the deformation amount of the blood vessel region for each frame can be accurately grasped. Therefore, even in the case of an ultrasonic image including few characteristic structures or an ultrasonic image in which speckles are dominant, it is possible to align blood vessel regions between frames with high accuracy and to obtain a high-quality and high-resolution blood flow image.

Although an example in which the present invention is applied to the ultrasonic diagnostic device has been described so far, the present invention may be applied to a modality (a medical information processing device) other than the ultrasonic diagnostic device. For example, similarly to the ultrasonic image, it is possible to obtain high-resolution blood flow information in a medical information processing device that acquires measurement data including tissue-derived information and blood-flow-derived information from a living body.

Subsequently, some embodiments of more specific embodiments of the present invention will be exemplarily described.

### <First Embodiment>

Fig. 1 is a block diagram illustrating an example of a hardware configuration of an ultrasonic diagnostic device according to the present embodiment. The ultrasonic diagnostic device 1 includes an ultrasonic probe 102, a probe connection unit 103, a transmission electric circuit 104, a reception electric circuit 105, a reception signal processing unit 106, an image processing unit 107, a display device 108, and a system control unit 109. The ultrasonic diagnostic device 1 is a system for transmitting a pulsed ultrasonic signal from the ultrasonic probe 102 to an object 100, receiving the ultrasonic signal reflected in a living body, and generating image information inside the object 100. An ultrasonic image such as a B-mode image, a blood flow image, or the like obtained by the ultrasonic diagnostic device 1 is used in various clinical examinations.

The ultrasonic probe 102 is an electronic scanning probe, and has a plurality of vibrators 101 arranged one-dimensionally or two-dimensionally at the tip thereof. The vibrator 101 is an electromechanical transducer that performs mutual conversion between an electric signal (a voltage pulse signal) and an ultrasonic wave (an acoustic wave). The ultrasonic probe 102 transmits ultrasonic waves from the plurality of vibrators 101 to the object 100, and receives reflected ultrasonic waves from the object 100 by the plurality of vibrators 101. The reflected acoustic wave reflects a difference in acoustic impedance in the object 100. Note that the reflected ultrasonic signal in a case where the transmitted ultrasonic pulse is reflected by a moving blood flow, the surface of a heart wall, or the like receives frequency shift depending on a velocity component with respect to the ultrasonic transmission direction of a moving body due to the Doppler effect.

The transmission electric circuit 104 is a transmission unit that outputs a pulse signal (a drive signal) to the plurality of vibrators 101. By applying pulse signals to the plurality of vibrators 101 with a time difference, ultrasonic waves having different delay times are transmitted from the plurality of vibrators 101, thereby forming a transmission ultrasonic beam. The direction and focus of the transmission ultrasonic beam can be controlled by selectively changing the vibrator 101 to which the pulse signal is applied (that is, the vibrator 101 to be driven) or changing the delay time (application timing) of the pulse signal. An observation region inside the object 100 is scanned by sequentially changing the direction and focus of the transmission ultrasonic beam. Furthermore, by changing the delay time of the pulse signal, a transmission ultrasonic beam, which is a plane wave (focus is distant) or a diffused wave (focus point is opposite to the ultrasonic transmission direction with respect to the plurality of vibrators 101), may be formed. Alternatively, the transmission ultrasonic beam may be formed using one vibrator or a part of the plurality of vibrators 101. The transmission electric circuit 104 generates a transmission ultrasonic wave having a predetermined transmission waveform in the vibrator 101 by transmitting a pulse signal having a predetermined drive waveform to the vibrator 101. The reception electric circuit 105 is a reception unit that inputs, as a reception signal, an electric signal output from the vibrator 101 that has received the reflected ultrasonic wave. The reception signal is input to the reception signal processing unit 106.

Operation of the transmission electric circuit 104 and the reception electric circuit 105, that is, transmission and reception of ultrasonic waves is controlled by the system control unit 109. For example, the system control unit 109 changes a position where a voltage signal or a transmission ultrasonic wave is formed according to each of generation of a B-mode image and generation of a blood flow image, which will be described later.

In the case of generating a B-mode image, a reception signal of a reflected ultrasonic wave obtained by scanning an observation region is acquired and used for image generation. In the case of generating a blood flow image, ultrasonic waves are transmitted and received a plurality of times on one or a plurality of scanning lines in the observation region, and reception signals of reflected ultrasonic waves of a plurality of frames are acquired and used for image generation, that is, extraction of blood flow information. The scanning for generating the blood flow image may be performed by a method of performing transmission and reception a plurality of times on one scanning line and then performing transmission and reception on the next scanning line, or may be a method of repeating transmission and reception once on each scanning line a plurality of times. In addition, in the generation of the B-mode image and the blood flow image, the number of scanning lines may be reduced, that is, a plane wave or a diffused wave may be transmitted to transmit an ultrasonic wave over a wide range of an observation region. In addition, the transmission angle of the plane wave or the diffused wave, the range of the observation region to which the plane wave or the diffused wave is transmitted, and the like may be changed to perform transmission and reception a plurality of times in a wide range of the observation region, and the reception signals may be added and used.

In the present specification, both an analog signal output from the vibrator 101 and digital data obtained by sampling (digitally converting) the analog signal are referred to as a reception signal without particular distinction. However, for the purpose of clearly indicating that the reception signal is digital data depending on the context, the reception signal may be referred to as reception data or measurement data.

The reception signal processing unit 106 is an image generation unit that generates image data based on the reception signal obtained from the ultrasonic probe 102. The image processing unit 107 performs image processing such as luminance adjustment, interpolation, and filter processing on the image data generated by the reception signal processing unit 106. The display device 108 is a display unit for displaying image data and various types of information, and includes, for example, a liquid crystal display, an organic EL display, or the like. The system control unit 109 is a control unit that integrally controls the transmission electric circuit 104, the reception electric circuit 105, the reception signal processing unit 106, the image processing unit 107, the display device 108, and the like.

### (Configuration of Reception Signal Processing Block)

Fig. 2 is a block diagram illustrating an example of a function of the reception signal processing unit 106. The reception signal processing unit 106 includes a reception signal storage unit 200, a phasing addition processing unit 201, a signal storage unit 202, a B-mode processing unit 203, a Doppler processing unit 204, a deformation amount calculation unit 205, a deformation correction unit 206, and a super-resolution processing unit 207.

The reception signal storage unit 200 stores a reception signal received by the reception electric circuit 105. Note that, depending on a device configuration and a type of the reception signal, the reception signal may not be stored in the reception signal storage unit 200, but may be stored in the signal storage unit 202 after the phasing addition processing unit 201 to be described later. In addition, the reception signal storage unit 200 may include a block common to the signal storage unit 202 to be described later, and may store the reception signal from the reception electric circuit 105 and the reception signal after the phasing addition processing unit 201.

The phasing addition processing unit 201 performs phasing addition or orthogonal detection processing on a reception signal obtained by the reception electric circuit 105, and stores the processed reception signal in the signal storage unit 202. The phasing addition processing is processing of forming a reception ultrasonic beam by changing a delay time and a weight for each vibrator 101 and adding reception signals of a plurality of vibrators 101, and is also referred to as Delay and Sum (DAS) beamforming. The orthogonal detection processing is processing of converting a reception signal into an in-phase signal (I signal) and an orthogonal signal (Q signal) in a baseband. The phasing addition processing and the orthogonal detection processing are performed based on various conditions (opening control, signal filter) of element arrangement and image generation input from the system control unit 109. The reception signal after the phasing addition processing and the orthogonal detection processing is stored in the signal storage unit 202. Here, an example of representative DAS beamforming has been described, but any processing may be used as long as the processing forms a reception ultrasonic beam, such as adaptive beamforming, model-based processing, and processing using machine learning.

The reception signal (digital data) obtained by the reception electric circuit 105 is also referred to as RAW data. The reception signal (digital data) obtained by the phasing addition processing unit 201 is also referred to as RF data. In the present embodiment, the RAW data and the RF data are an example of measurement data acquired based on an ultrasonic signal reflected in a living body, and the reception electric circuit 105 and the phasing addition processing unit 201 are an example of a first acquisition unit that acquires measurement data.

The B-mode processing unit 203 performs envelope detection processing, logarithmic compression processing, and the like on the reception signal for generating the B-mode image stored in the signal storage unit 202, and generates image data in which signal intensity at each point in an observation region is represented by luminance intensity.

A processing content of the Doppler processing unit 204 will be described in detail. The Doppler processing unit 204 extracts blood flow information based on the Doppler effect of a target object, which is located within the scanning range, by performing frequency analysis on the reception signal for blood flow image generation, which is stored in the signal storage unit 202. In the present embodiment, an example in which a target object is blood will be mainly described, but the target object may be an object such as a body tissue or a contrast medium. Examples of the blood flow information include at least one of a velocity, a dispersion value, and a power value. In addition, the Doppler processing unit 204 may obtain blood flow information at one point (one position) in the object, or may obtain blood flow information at a plurality of positions in the depth direction. In addition, the Doppler processing unit 204 may obtain the blood flow information at a plurality of time points in time series so that a temporal change of the blood flow information can be displayed.

In the generation of the blood flow image by the Doppler method, reception signal data strings of a plurality of frames are acquired in the time direction for the same measurement position. The Doppler processing unit 204 applies a moving target indicator (MTI) filter to the reception signal data string. As a result, information (clutter component) derived from a tissue that is stationary between frames or a tissue with small motion is reduced, and information (blood flow component) derived from a blood flow is extracted. Then, the Doppler processing unit 204 calculates blood flow information such as the velocity of the blood flow, the dispersion of the blood flow, and the power of the blood flow from the blood flow component. Data representing the calculated blood flow information in the form of a two-dimensional image is referred to as a blood flow image.

As the MTI filter, a filter having a fixed filter coefficient, such as a Butterworth infinite impulse response (IIR) filter or a polynomial regression filter, may be used. The MTI filter may be an adaptive filter that changes a coefficient depending on an input signal using eigenvalue decomposition, singular value decomposition, or the like. Alternatively, the Doppler processing unit 204 may decompose the reception signal into one or a plurality of bases using eigenvalue decomposition, singular value decomposition, or the like, extract only a specific basis, and remove a clutter component. In addition, the Doppler processing unit 204 may obtain a velocity vector for each coordinate in the image by using a method such as a vector Doppler method, a speckle tracking method, or a vector flow mapping method to obtain a blood flow vector representing the magnitude and the direction of a blood flow. In addition to the method exemplified here, any method may be used as long as the method is a method capable of extracting or emphasizing the blood-flow-derived information included in a reception signal (measurement data) or removing or reducing the tissue-derived information.

The deformation amount calculation unit 205 uses blood flow images of a plurality of frames, output by the Doppler processing unit 204, to calculate a difference in the position and shape of a blood vessel region between the frames (a deformation amount of the blood vessel region). In the present embodiment, the blood flow image is an example of blood flow data in which the blood-flow-derived information is extracted or emphasized, and the Doppler processing unit 204 and the deformation amount calculation unit 205 are an example of the second acquisition unit that acquires the deformation amount between a plurality of frames based on the blood flow image (blood flow data).

Prior to the processing of calculating the deformation amount, the deformation amount calculation unit 205 may perform pre-processing for emphasizing or clarifying a blood vessel structure in the blood flow image on the blood flow image. As the pre-processing, for example, a filter that extracts or emphasizes a vascular-shaped (for example, a columnar shape, a linear shape, a mesh shape, or the like) structure included in the image may be applied to the blood flow image. Conversely, the blood vessel structure may be relatively emphasized by applying, to the blood flow image, a filter that removes or reduces a background in the image (a tissue portion other than the blood vessel structure) and noise in the background. For example, the deformation amount calculation unit 205 may perform multi-resolution decomposition such as pyramid transformation or wavelet transformation on the blood flow image, may apply an appropriate filter to each image, and then may perform inverse transformation of the multi-resolution decomposition. As a result, a structure different from the background is clearly extracted.

In addition, the deformation amount calculation unit 205 may emphasize a linear structure (that is, a blood vessel portion) in the image by applying line emphasis processing by eigenvalue analysis of the Hessian matrix to the blood flow image. Further, the deformation amount calculation unit 205 may extract only the blood vessel structure from the blood flow image by image extraction processing such as template matching using a template of the blood vessel structure.

As a method of calculating the deformation amount, rigid body registration may be used, or non-rigid body registration may be used. Alternatively, the deformation amount may be calculated using a learned model by deep learning or the like. In addition, the present invention is not limited thereto, and any method may be used as long as a deformation amount for correcting the position and shape changed by body motion or the like between frames can be calculated for the blood flow image. A user may select a desired method from a plurality of calculation methods prepared in advance.

The rigid body registration is a method in which a target object (a blood vessel in the present embodiment) is assumed to be a rigid body and registration is performed using a transformation function including elements of only translation and rotation in an image. Further, the rigid body registration has an advantage in that calculation cost is small and high-speed processing is possible. In the case of rigid body registration, one deformation amount may be calculated for the image (that is, the entirety of an image is translated and rotated), or the image may be divided into a plurality of regions, and the deformation amount may be calculated for each region. In the latter case, since the movement amount and the rotation amount may be different for each region, boundary portions are smoothly connected to each other so that no artifact occurs at a boundary between adjacent regions. For example, an overlap having a predetermined width may be formed at a boundary portion, and pixel values of two regions may be averaged or blended at the overlapping portion. A blending ratio may be gradually changed between two regions. In addition, the deformation amount may be gradually changed by interpolating the deformation amounts of two regions in the overlapping portion.

The non-rigid body registration includes a method of using affine transformation such as scaling and shearing on the assumption that a target object (a blood vessel in the present embodiment) is a non-rigid body, a method of deforming an image with a deformation element such as local non-linear deformation. The non-rigid body registration is also referred to as deformable image registration (DIR). Note that, since movement is often accompanied by non-linear deformation in a living body image such as a blood flow image, it is desirable to use non-rigid body registration. Algorithms used for the non-rigid body registration include, for example, a free form deformation (FFD) method and a Demons algorithm. The FFD method is a method of deforming an image using a B-spline function by setting control grid points (sampling points) for an image to be deformed and obtaining a control point matrix from a target movement amount. The Demons algorithm is a method of generating a deformation vector based on a signal difference or a gradient at the same position between two images.

In the case of using deep learning, for example, a learned model that is trained to output, when a blood flow image is input to the learned model, a deformation amount in the blood flow image may be used. In this case, reference data (the position and shape of a blood vessel serving as a reference) may be fixed (that is, learned in advance in the learned model), or a reference image may be given as an input together with a blood flow image to be processed. When machine learning of a model is performed, supervised learning using a set of a reference image, a blood flow image after deformation, and a deformation amount (a true value) as training data may be performed.

Alternatively, a learned model that is trained to output, when a blood flow image is input to the learned model, a corrected image in which blood vessel deformation has been corrected may be used. In this case, the learned model has two functions of the deformation amount calculation unit 205 and the deformation correction unit 206. In the case of such a learned model as well, reference data (the position and shape of a blood vessel serving as a reference) may be fixed (that is, learned in advance in the learned model), or a reference image may be given as an input together with a blood flow image to be processed. When machine learning of a model is performed, supervised learning using a set of a reference image (a true value) and an image before correction as training data may be performed.

The deformation correction unit 206 corrects the position and shape of a blood vessel in an image, that is, aligns the blood vessel between frames based on a deformation amount output by the deformation amount calculation unit 205. The deformation correction unit 206 may apply correction to a reception signal (measurement data) stored in the reception signal storage unit 200 or the signal storage unit 202, or may apply correction to a blood flow image (blood flow data) obtained by the Doppler processing unit 204. Alternatively, the deformation correction unit 206 may apply correction to secondary data (for example, an image obtained by averaging blood flow images of a plurality of frames, or the like) generated from the reception signal or the blood flow image. Note that, in the present embodiment, after the deformation correction unit 206 corrects an image, the corrected image is transferred to the super-resolution processing unit 207 in the subsequent stage. However, the deformation correction may not be performed explicitly, and the position of a feature point may be corrected together at the time of integration processing in the super-resolution processing unit 207. In the latter case, since the super-resolution processing unit 207 also serves as the deformation correction unit 206, the deformation correction unit 206 in Fig. 2 may be omitted.

The super-resolution processing unit 207 generates super-resolution blood flow image data, which is a blood flow image having improved resolution, from blood flow image data for a plurality of frames aligned (blood vessel deformation is corrected) by the deformation correction unit 206. A method of acquiring a super-resolution blood flow image will be described in the description of a processing flow. In the present embodiment, the deformation correction unit 206 and the super-resolution processing unit 207 are an example of a generation unit that generates display image data.

The image data output from the B-mode processing unit 203, the Doppler processing unit 204, and the super-resolution processing unit 207 is processed by the image processing unit 107 illustrated in Fig. 1 and then is finally displayed on the display device 108. The respective pieces of image data may be displayed in a superimposed manner or may be displayed in parallel, or only a part of the image data may be displayed.

The reception signal processing unit 106 may include one or more processors and a memory. In this case, the function of each of the units 201 to 207 illustrated in Fig. 2 is implemented by a computer program. For example, the function of each of the units 201 to 207 can be provided by a CPU reading and executing a program stored in the memory. In addition to the CPU, the reception signal processing unit 106 may include a processor (GPU, FPGA, and the like) in charge of calculation of the B-mode processing unit 203, the Doppler processing unit 204, the deformation amount calculation unit 205, the deformation correction unit 206, and the super-resolution processing unit 207. The memory may include a memory for non-temporarily storing a program, a memory for temporarily storing data such as a reception signal, a working memory used by the CPU, and the like.

The overall configuration of the ultrasonic diagnostic device 1 according to the first embodiment has been described above. Next, a processing flow for acquiring a super-resolution blood flow image will be described.

### (Processing Flow for Obtaining Super-Resolution Blood Flow Image)

A processing flow for acquiring a super-resolution blood flow image according to the first embodiment will be described with reference to Fig. 3.

A measurement position is set so as to include a region where blood flow information is desired to be observed, and ultrasonic transmission/reception is repeatedly performed in a state of being fixed at the same measurement position to acquire a reception signal including a data string of a plurality of frames in a time direction (S310). In general, the number of frames of the reception signal when the super-resolution blood flow image according to the present embodiment is generated is larger than that when a normal Doppler image is generated. For example, the number of frames (the number of packets) of the reception signal when the normal Doppler image is generated is about 5 to 20 frames, whereas the number of frames of the reception signal when the super-resolution blood flow image is generated may be at least 100 frames or more, and preferably, may be about several hundred to several tens of thousand frames. A frame rate of the ultrasonic diagnostic device 1 is about several hundred Hz to several thousand Hz depending on a model and a measurement method. Therefore, the measurement time of the reception signal for generating the super-resolution blood flow image is about several hundred msec to several tens of sec. A reception signal including a data string of a plurality of frames is subjected to phasing addition and orthogonal detection processing by the phasing addition processing unit 201, and the reception signal is stored in the signal storage unit 202. Further, one frame in the data string may be a reception signal obtained by performing ultrasonic wave transmission/reception one time so as to include an observation region, or a frame obtained by adding a reception signal transmitted and received a plurality of times so as to include an observation region may be used as one frame. For example, a plane wave or a diffused wave is transmitted so as to include an observation region, and a plurality of reception signals, the transmission angles of which have been changed, are added to obtain data of one frame.

The Doppler processing unit 204 reduces a clutter component by applying an MTI filter to the reception signal including the data string of the plurality of frames, and extracts a component derived from the blood flow to generate a blood flow image of the plurality of frames (S320). A method of setting a frame when the blood flow image is generated is illustrated in Fig. 4. By extracting a part of the data string of the reception signal and designing the MTI filter, the same number of blood flow images (before averaging) as the number of extracted frames (the number of packets) can be obtained. These blood flow images are averaged (addition average, square root of the sum of squares, and the like) to obtain a blood flow image (after averaging). In a normal Doppler, this blood flow image (after averaging) is displayed. By changing a frame to be extracted in the time direction, a blood flow image can be further obtained. At this time, frames to be extracted may overlap each other or may not overlap each other. An example of the obtained blood flow image is illustrated in Fig. 5A. Note that parameters of the MTI filter (cutoff frequency, number of packets and number of overlaps of frames to be extracted, and the like) may be adjustable automatically or by a user depending on the properties of a reception signal, an object to be measured, and the like.

The deformation amount calculation unit 205 calculates a deformation amount of a blood vessel region, generated by body motion or the like between the frames, from the blood flow image (S330). In the calculation of the deformation amount, a blood flow image (after averaging) may be used, or a blood flow image (before averaging) may be used. Next, the deformation correction unit 206 corrects the corresponding blood flow image based on the calculated deformation amount such that the blood flow (blood vessel) positions are aligned between the frames to be subjected to integration processing in S360 to be described later (S340).

An example of correction with non-linear deformation in a blood flow image will be described with reference to Figs. 6A to 6C. Figs. 6A to 6C illustrate time-series blood flow images, and the respective blood flow images are blood flow images having different frames (time points). Broken lines in Figs. 6B and 6C represent blood flow (blood vessel) positions in Fig. 6A. The blood flow (blood vessel) position is not only translated or rotated but also locally non-linearly deformed. When such non-linear deformation occurs, the above-described non-rigid body registration or the correction by a learned model may be applied. For example, the deformation amount calculation unit 205 selects a reference image (a reference frame) from among the time-series blood flow images, and calculates relative displacement or deformation with respect to the position or shape of a blood vessel region of a reference frame for blood flow images of other frames. In the illustrated example, Fig. 6A is set as a reference frame, and a deformation amount is obtained for each of the frames of Figs. 6B and 6C. When locally non-linear deformation is generated, a local deformation amount is obtained for each of a plurality of coordinates in a blood flow image. Then, the deformation correction unit 206 applies correction by non-rigid body registration to an image of each frame so as to cancel the deformation amount, so that the position and shape of a blood vessel region in the blood flow image in Figs. 6B and 6C coincide with those of a blood vessel region in the blood flow image in Fig. 6A.

The reference frame at the time of calculating the deformation amount may be only one frame in the entirety of a data string, the reference frame may be changed (that is, a plurality of reference frames are provided) according to the position in the time direction, or one previous frame in the adjacent frame may be used as the reference frame. The reference frame may be arbitrarily selected from among the data strings, and for example, any one of the first frame, the intermediate frame, and the last frame may be used as a reference frame. However, among the plurality of frames forming the data string, a frame including the most blood-flow-derived information, for example, a frame in which the most blood vessels (blood flows) are visualized may be selected as a reference frame. Selecting a reference frame in this manner not only enables alignment of a larger number of blood flows (blood vessels) in the image, but also improves accuracy of the alignment. Figs. 7A to 7B are examples of blood flow images, and in Fig. 7A, a blood flow divided into two is clearly visualized, whereas in Fig. 7B, there is much noise and the entirety of the blood flow is unclear, and in Fig. 7C, a thin blood flow is unclear. In the case of such a data string, the image of Fig. 7A having the best quality may be selected as a reference frame. As an index for evaluating that a large amount of blood flow (blood vessel) is visualized, for example, a signal-to-noise ratio (SNR) or a contrast-to-noise ratio (CNR) of an image may be used. Alternatively, an average luminance value of an image, a histogram, the number of pixels exceeding a predetermined threshold value, the number of linear structures, or the like may be used as an evaluation index.

The blood flow image used in the calculation of the deformation amount in S330 may be either a blood flow image before averaging or after averaging. However, in the case of the blood flow image after averaging, the SNR of the blood flow structure is better, and accuracy of the deformation amount calculation is higher. In addition, the blood flow image to which the correction used in S340 is applied may be either a blood flow image before averaging or after averaging. However, since the blood flow image before averaging has a larger pixel value difference in the blood flow, it is easy to extract the local maximum value of the blood flow image in S350 to be described later. When the blood flow image before averaging is corrected by calculating the deformation amount in the blood flow image after averaging, the blood flow image (before averaging) may be a part or all of the packet, and a frame used for the integration processing in S360 to be described later is corrected.

The super-resolution processing unit 207 extracts a feature point in the blood-flow-derived information from each corrected blood flow image (S350). In the blood flow image according to the present embodiment, the blood-flow-derived information is expressed as a pixel value (luminance; concentration), and a pixel having more (stronger) blood-flow-derived information (that is, a pixel corresponding to a presence location of the blood flow) has a larger pixel value. Therefore, the super-resolution processing unit 207 extracts a point (pixel) having a large pixel value as a feature point from the blood flow image. By the operation in S350, an image in which only points where the blood-flow-derived information is particularly strongly expressed in the blood flow image are extracted as illustrated in Fig. 5B is obtained.

A lower limit and an upper limit of a range (hereinafter, referred to as a "predetermined range" or an "extraction range") of pixel values from which feature points are extracted may be fixed values, or may be dynamically set according to a pixel value distribution (histogram) or a maximum pixel value of the blood flow image. For example, the maximum pixel value of the blood flow image may be set as the upper limit, and a value obtained by multiplying the maximum pixel value by a predetermined coefficient (for example, 0.8) may be set as the lower limit. At this time, if the coefficient is set to 1.0, only the pixel having the maximum pixel value in the blood flow image is extracted, and adjustment can be performed such that a larger number of pixels are extracted as the coefficient is reduced. Alternatively, an extraction range may be set so as to include the top N (N is an arbitrarily set integer) pixels of the histogram.

The super-resolution processing unit 207 may extract a pixel having the local maximum value as a feature point. For example, a blood flow image is divided into a plurality of local regions, and a feature point is extracted for each of the local regions. At this time, the super-resolution processing unit 207 may fix an extraction range of each local region, or may set an extraction range for each local region according to the local maximum pixel value. Furthermore, a pixel having a larger pixel value than an adjacent pixel (for example, when a pixel value of a central pixel becomes maximum in a local region of 3 pixels × 3 pixels), that is, a pixel having the maximum pixel value may be extracted, and the local maximum value may be extracted. Here, an example of a representative method of extracting the local maximum value has been described, but any method may be used as long as the method is processing of extracting the local maximum value. A larger number of pixels can be extracted as feature points by setting an extraction range for each local region as compared with a case in which a uniform extraction range is set for the entirety of an image, and the number of pixels to be used in the integration processing in S360 to be described later can be increased. Note that, in a case where the extraction range is set for each local region, not only pixels in the blood flow but also pixels in the tissue (that is, noise) may be extracted. However, since the pixel value of the pixel (noise) in the tissue tends to be relatively smaller than that of the pixel in the blood flow, the influence of the pixel in the tissue is reduced by integrating pixel groups extracted in S360 to be described later with the pixel value. Therefore, in most cases, a super-resolution image having an SNR equal to or higher than that of an original blood flow image is finally obtained, and there is no particular problem.

Alternatively, before performing the integration processing in S360, the super-resolution processing unit 207 may perform processing of actively removing pixels (noise) in the tissue from the pixel group extracted in S350. Specifically, a threshold value may be set for the pixel value distribution (histogram) of the pixel group extracted in S350, and a pixel having a pixel value smaller than the threshold value may be removed as noise. The threshold value may be set by a user. For example, the threshold value may be input or selected on a setting screen displayed on the display device 108, or may be designated by a GUI such as a slider bar. At this time, the pixel value distribution (histogram) of the pixel group extracted in S350 may be displayed on the setting screen, and further, auxiliary information such as the position of Nσ and the position of a valley of the pixel value distribution may be displayed. Such display can assist the user in selecting a threshold value. Alternatively, the super-resolution processing unit 207 may automatically set the threshold value. In this case, a fixed threshold value may be used, or the threshold value may be dynamically determined based on variance, bimodality, outlier, or the like of the pixel value distribution. Alternatively, M (M is any set integer) lower pixels may be mechanically removed as noise from a pixel group having a small pixel value among the extracted pixel groups.

In addition, the pixel value of the blood flow image varies depending on the depth from the body surface due to the influence of attenuation by a living body or diffusion attenuation of ultrasonic waves. Therefore, in the processing of S350, the super-resolution processing unit 207 may set the above-described extraction range according to the "depth from the body surface" for each local region in the blood flow image. That is, when the blood flow image includes a first local region and a second local region having different depths from the body surface, an extraction range of the first local region and an extraction range of the second local region are made different in consideration of the depth from the body surface. For example, in a case where the second local region is deeper from the body surface than the first local region and the pixel value of the second local region becomes smaller than that of the first local region due to the influence of the attenuation of the ultrasonic wave, the extraction range of the second local region may be wider (for example, the lower limit may be lowered) than the extraction range of the first local region. Alternatively, instead of changing the extraction range, processing of correcting biological attenuation or diffusion attenuation may be applied to the blood flow image before processing of extracting a feature point.

Alternatively, the super-resolution processing unit 207 may integrate blood flow images of a plurality of frames to generate a blood flow image with an improved SNR, and then extract feature points in S350 from the integrated blood flow image. Further, the super-resolution processing unit 207 may not use the blood flow images of all the frames for the feature point extraction processing in S350 and the integration processing in S360. For example, a frame having a poor SNR may be excluded from processing targets in S350 and S360.

In addition, the super-resolution processing unit 207 may apply the feature point extraction processing in S350 after performing processing of increasing (up-converting) the resolution of the blood flow image. At this time, the blood flow image itself may be up-converted, or the original reception signal may be up-converted, and the blood flow image may be generated using the reception signals of a plurality of frames after up-conversion. An up-conversion method is arbitrary, and an interpolation pixel may be generated by, for example, a nearest-neighbor, linear interpolation, spline interpolation, or the like. Alternatively, up-conversion may be performed by applying a filter generated by machine learning. Since the resolution (the number of pixels) of the super-resolution blood flow image obtained by the integration processing in S360 is the same as the resolution (the number of pixels) of an original image used to extract the feature point, the effect of improving resolving power (resolution) by the super-resolution processing is small when the resolution of the original image is low. Therefore, by increasing the resolution (the number of pixels) of the original image in advance, the resolving power of the final super-resolution blood flow image can be increased. Further, in images obtained using a convex probe or a sector probe, the pixel size (resolution) differs between a deep region and a shallow region in the image. By increasing the resolving power of the shallow region in advance by up-conversion, the effect of improving the resolving power of the final super-resolution blood flow image can also be expected.

Since only a portion having a high pixel value is extracted in the feature point extraction in S350, assuming that a point spread function is the Gaussian distribution, only a portion (peak) having a high sharpness at the center of the distribution is extracted. In general, it is known that amplitude distribution characteristics of an ultrasonic reception signal from a uniform scattering medium can be roughly approximated by a probability distribution called the Rayleigh distribution illustrated in Fig. 8. The extraction of a portion having a high pixel value in S350 means that a portion having a small probability but a large amplitude value in the Rayleigh distribution is extracted from a large number of reflection signals derived from red blood cells, so that only a small number of reflection signals derived from red blood cells are spatially sparse. Only a point or a pixel in a minute range is extracted from an image of one frame, but a portion having a high pixel value moves between the frames. Therefore, by extracting a feature point from each of a large number of frames, a feature point can be comprehensively obtained from almost the entire region of the blood flow range.

Finally, the super-resolution processing unit 207 generates a super-resolution blood flow image by integrating pixel values of the feature points extracted from a large number of frames (S360). Since pixels having a large pixel value are concentrated in a blood flow range by the integration processing, in the super-resolution blood flow image, the blood flow is imaged (visualized) with higher resolution and clarity as compared with an original blood flow image. The super-resolution processing unit 207 displays the generated super-resolution blood flow image on the display device 108.

Note that images of all frames stored in the signal storage unit 202 may be used for the feature point extraction and integration processing to generate one super-resolution blood flow image, or only frames in a partial time range may be used for generation of the super-resolution blood flow image. In the latter case, a user may select a time range used for generation of the super-resolution blood flow image. In addition, a plurality of super-resolution blood flow images in time series may be generated while sequentially shifting a time range used for generation of the super-resolution blood flow image. Furthermore, moving image display of the super-resolution blood flow image may be performed by sequentially displaying a plurality of super-resolution blood flow images on the display device 108. Alternatively, a plurality of super-resolution blood flow images may be displayed side by side on the display device 108 so that temporal changes can be compared.

According to the configuration of the present embodiment described above, even in a case where there is movement or deformation between frames, it is possible to generate a high-resolution and high-quality super-resolution blood flow image by accurately adjusting the blood flow positions.

### <Second Embodiment>

In the first embodiment, a feature point is extracted from a blood flow image after deformation correction. In the second embodiment, a plurality of feature separation images having different image features are generated from the blood flow image after deformation correction, and feature points are extracted from the respective feature separation images.

A feature amount space formed by all image features included in a blood flow image is considered. For example, by separating a part of a subspace from the feature amount space and reconstructing an image only using the image features included in the subspace, it is possible to generate an image (referred to as a feature separation image) obtained by separating only a part of the image features in the original blood flow image. At this time, by making image features of interest (that is, the subspace separated from the feature amount space) different, a plurality of feature separation images having different image features can be generated from one blood flow image. Note that the subspaces of the respective feature separation images may be completely separated, or the subspaces may partially overlap each other.

A device configuration may be the same as that of the first embodiment. A processing flow for acquiring a super-resolution blood flow image according to the second embodiment will be described with reference to Fig. 9. The processing from S710 to S740 is similar to that from S310 to S340 of the first embodiment. The super-resolution processing unit 207 generates a plurality of feature separation images having different image features from respective blood flow images subjected to deformation correction (S750).

Fig. 10 illustrates a frame setting method when a plurality of feature separation images are generated from the blood flow image subjected to deformation correction. When acquiring the blood flow image after deformation correction, the super-resolution processing unit 207 generates a plurality of feature separation images having different image features of interest from the respective blood flow images. Although Fig. 10 illustrates a case in which the deformation correction is performed on the blood flow image before averaging, the deformation correction may be performed on the blood flow image after averaging as in the first embodiment. In this case, a plurality of feature separation images may be generated from the blood flow image after deformation correction (after averaging).

Here, an effect of dividing a blood flow image into a plurality of feature separation images will be described. Generally, in a blood flow image, between a "thick blood vessel having a high flow velocity" and a "thin blood vessel having a low flow velocity," the thick blood vessel having a high flow velocity has a larger pixel value. Therefore, when a pixel group having a pixel value in a predetermined range is extracted from a blood flow image in which a thick blood vessel having a high flow velocity and a thin blood vessel having a low flow velocity are adjacent to each other, there is a possibility that pixels of the thin blood vessel having a low flow velocity are not extracted. It is also conceivable to expand an extraction range of a pixel value to be extracted as a feature point so that a blood vessel having a small pixel value, that is, a blood vessel having a low flow velocity is also extracted. However, considering extraction with respect to the Gaussian distribution described above, since the range of the extracted distribution is widened, the effect of improving resolution is reduced. In order to solve this problem, a feature separation image may be generated using an image feature related to the flow velocity of a blood flow. That is, a plurality of feature separation images having different flow velocity ranges, such as a first feature separation image having an image feature corresponding to a first flow velocity range and a second feature separation image having an image feature corresponding to a second flow velocity range, are generated from an original blood flow image. Then, an appropriate extraction range is set for each feature separation image, and a feature point is extracted from each feature separation image. By such a method, both pixels of a thick blood vessel having a high flow velocity and pixels of a thin blood vessel having a low flow velocity can be extracted as feature points.

Specifically, the discrete Fourier transform is performed on a change in the pixel value of each pixel in the time direction by using a part of the frames among the blood flow images of the plurality of frames. The discrete Fourier transform is a sinusoidal basis and represents a frequency component. Therefore, division can be performed by the discrete Fourier transform for each frequency component of an interframe change in each pixel of the blood flow image, that is, for each flow velocity component, and it is possible to generate a feature separation image having blood flow information in a flow velocity range corresponding to each basis. The feature separation image having the blood flow information in the flow velocity range corresponding to each basis may be an image in a frequency domain after the discrete Fourier transform, or may be an image in a time domain by performing inverse discrete Fourier transform on an image in the frequency domain. By changing a part of the frames used for the discrete Fourier transform among the blood flow images of the plurality of frames in the time direction, it is possible to obtain time-series different feature separation images corresponding to the respective basis. In this case, the blood flow image is separated into a plurality of feature separation images by focusing on image features (frequency components of temporal changes of pixel values) related to the flow velocity, that is, based on the flow velocity. By increasing the number of separations, that is, the number of frames used for the discrete Fourier transform, the flow velocity range corresponding to each basis is reduced, so that the blood flow image can be separated for each finer flow velocity range. Note that, as long as the basis can be separated for each flow velocity range, a method other than the discrete Fourier transform based on a sine wave may be used, and the blood flow image may be separated into images for each flow velocity range by polynomial regression such as Legendre polynomial. Furthermore, in a case where an energy level (for example, standard deviation of a pixel value) of a pixel value of a certain blood vessel is close to the energy of noise, the noise energy is dispersed to each basis by generating the feature separation image separated for each flow velocity by the processing in S750. As a result, the noise energy is reduced as compared with image information of a blood flow component, so that the signal-to-noise ratio (SNR) or the contrast-to-noise ratio (CNR) of each feature separation image can be improved. Therefore, there is also an advantage in that a blood vessel that cannot be distinguished from noise in the blood flow image can be depicted in the feature separation image. Furthermore, more feature points can be extracted by the processing in S760 to be described later, and more feature points can be extracted with the same number of frames. As a result, the quality of the final super-resolution blood flow image can be improved, and the super-resolution blood flow image having the same quality can be generated from a smaller number of frames than that in the first embodiment, so that the measurement time required to generate the super-resolution blood flow image can be shortened.

An image feature is not limited to a feature related to a flow velocity. For example, a feature separation image may be generated by focusing on an image feature related to the shape or thickness of a blood vessel. That is, extracted blood vessel diameters are different in the respective bases. For example, a spatial frequency of each blood flow image is calculated using two-dimensional Fourier transform. A high spatial frequency, that is, a spatially fine structure, is an image feature of a thin blood vessel, and a low spatial frequency, that is, a spatially coarse structure, is an image feature of a thick blood vessel. Therefore, for example, by extracting only a spatial frequency component corresponding to a desired blood vessel diameter from the two-dimensional Fourier transform of a blood flow image and performing the inverse Fourier transform, a feature separation image having an image feature corresponding to the desired blood vessel diameter can be generated. Then, by appropriately changing the spatial frequency component to be extracted, for example, a plurality of feature separation images corresponding to different blood vessel diameters, such as a feature separation image corresponding to a blood vessel diameter of 1 mm to 2 mm and a feature separation image corresponding to a blood vessel diameter of 2 mm to 3 mm, can be obtained. In addition, by applying line emphasis processing by eigenvalue analysis of the Hessian matrix to a blood flow image, luminance and contrast of a vascular-shaped (a linear shape, a columnar shape) structure having a desired thickness are selectively emphasized, so that a feature separation image in which an image feature corresponding to a desired blood vessel diameter is emphasized may be generated. In this case as well, a plurality of feature separation images corresponding to different blood vessel diameters can be generated by appropriately changing the thickness to be emphasized by the line emphasis processing. In addition, similarity search processing such as template matching may be used. For example, a similarity map (two-dimensional map representing similarity with a template image for each local region) is generated by searching the entirety of a blood flow image using a template image of a blood vessel pattern having a desired thickness. This similarity map can be regarded as a two-dimensional image filter in which a larger coefficient is set for a pixel corresponding to the blood vessel diameter of the template image. Therefore, by multiplying the blood flow image by the similarity map, a feature separation image in which an image feature corresponding to the blood vessel diameter of the template image is emphasized can be obtained. In this case, a plurality of feature separation images corresponding to different blood vessel diameters can be generated by preparing a plurality of template images having different blood vessel diameters. Other than the method described herein, any method may be used to generate the feature separation image as long as the method selectively emphasizes or extracts the image features corresponding to the thickness and shape of the blood vessel.

The feature separation image may be generated based on two image features of "temporal change and spatial structure", that is, "flow velocity and shape". For example, the discrete Fourier transform (three-dimensional discrete Fourier transform) is performed on a part of the frames among the blood flow images of the plurality of frames in both the time direction of the pixel value of each pixel and the spatial direction in the image. Then, by extracting only a spatial frequency component corresponding to a desired flow velocity range and corresponding to a desired blood vessel diameter and performing the inverse Fourier transform, it is possible to generate a feature separation image having an image feature corresponding to a desired flow velocity range and a desired blood vessel diameter. By appropriately changing the spatial frequency component to be extracted, it is possible to obtain a plurality of feature separation images having different combinations of the flow velocity range and the blood vessel diameter. Alternatively, such implementation can be performed by converting individual frames of a part of the frames among the blood flow images of the plurality of frames into a one-dimensional vector and using singular value decomposition for matrix data in which the respective frames are arranged in the column direction. As a result, images of the blood vessels having spatially different sizes can be further divided into images having different flow velocity ranges.

Feature separation images may be generated focusing on image features associated with the direction of a blood flow. The velocity vector for each coordinate in the image is obtained by using a method such as a vector Doppler method, a speckle tracking method, or a vector flow mapping method, in addition to the blood flow image by the Doppler method. The direction of this velocity vector represents the direction of a blood flow. In order to generate the feature separation image corresponding to the desired blood flow direction, for example, a matching map indicating a degree of matching between a desired blood flow direction and a velocity vector for each coordinate is generated. This matching map can be regarded as a two-dimensional image filter in which a larger coefficient is set for coordinates having a velocity vector closer to a desired blood flow direction. Therefore, by multiplying the blood flow image by the matching map, a feature separation image in which an image feature corresponding to a desired blood flow direction is emphasized can be obtained. By generating the matching map for each blood flow direction and multiplying the blood flow image by the matching map, a plurality of feature separation images corresponding to different blood flow directions can be generated.

The super-resolution processing unit 207 extracts a feature point from each of the plurality of feature separation images generated in S750 (S760). In the first embodiment, a target is a blood flow image subjected to deformation correction, whereas in the second embodiment, a feature separation image generated from a blood flow image is a target. A method of determining a feature point extraction range may be the same as that in the first embodiment, but an appropriate extraction range is determined for each feature separation image.

Finally, the super-resolution processing unit 207 generates a super-resolution blood flow image by integrating pixel values of the feature points extracted from a large number of feature separation images (S760). The super-resolution processing unit 207 displays the generated super-resolution blood flow image on the display device 108.

All the feature separation images generated from the blood flow images of all the frames stored in the signal storage unit 202 may be used for feature point extraction and integration processing to generate one super-resolution blood flow image, or only frames in a partial time range may be used for generation of the super-resolution blood flow image. In the latter case, a user may select a time range used for generation of the super-resolution blood flow image. In addition, a plurality of super-resolution blood flow images in time series may be generated while sequentially shifting a time range used for generation of the super-resolution blood flow image. Furthermore, moving image display of the super-resolution blood flow image may be performed by sequentially displaying a plurality of super-resolution blood flow images on the display device 108. Alternatively, a plurality of super-resolution blood flow images may be displayed side by side on the display device 108 so that temporal changes can be compared.

All the feature points extracted from the plurality of feature separation images corresponding to different image features may be simply integrated, only a part of the feature points may be selected and integrated, or the feature points may be integrated with a weight corresponding to the image features. For example, if all feature points extracted from a plurality of feature separation images having different flow velocity ranges are simply integrated, all blood vessels (blood flows) from a blood vessel having a low flow velocity to a blood vessel having a high flow velocity can be imaged. When only the feature points of a feature separation image corresponding to a specific flow velocity range are selected or the weight of the feature points is increased and then the weighted feature points are integrated, an image in which a blood vessel in the specific flow velocity range is emphasized can be obtained. A GUI for arbitrarily selecting or setting a range of image features (feature separation images) used for integration and a weight according to the image features may be provided to the user. By using such a GUI, it is possible to generate an appropriate super-resolution blood flow image according to an object (the thickness, shape, flow velocity, direction, and the like of a blood vessel) to be observed.

### <Third Embodiment>

In the third embodiment, deformation correction is applied to a reception signal instead of a blood flow image. By generating a blood flow image from a reception signal after deformation correction and using the blood flow image for super-resolution processing, a high-resolution and high-quality super-resolution blood flow image can be generated.

A device configuration may be the same as that of the first embodiment. A processing flow for acquiring a super-resolution blood flow image according to the third embodiment will be described with reference to Fig. 11. The processing from S910 to S930 is similar to that from S310 to S330 of the first embodiment. That is, in the third embodiment as well, the deformation amount calculation unit 205 calculates a deformation amount of a blood vessel region caused by body motion or the like between frames from a blood flow image (S930). The blood flow image used in the calculation of the deformation amount may be either a blood flow image before averaging or after averaging. However, in the case of the blood flow image after averaging, the SNR of a blood flow structure is better, and accuracy of deformation amount calculation is higher. A specific method of calculating the deformation amount is as described in the first embodiment.

Next, the deformation correction unit 206 corrects, based on the deformation amount calculated from the blood flow image in S930, a reception signal corresponding to the blood flow image (S940). A specific correction method may be the same as that described in the first embodiment, and any of correction by rigid body registration, correction by non-rigid body registration, and correction by a learned model may be used.

Next, the Doppler processing unit 204 removes clutter from the reception signal subjected to deformation correction to generate a plurality of blood flow images (S950). A method of generating the blood flow image may be the same as that described in the first embodiment.

Fig. 12 illustrates a method of setting a frame when the blood flow image is generated from the reception signal subjected to deformation correction. Parameters (cutoff frequency, number of packets to be extracted, number of overlaps, and the like) of the MTI filter may be adjustable automatically or by a user depending on the properties of a reception signal, an object to be measured, and the like. The parameters of the MTI filter may be the same or changed in S920 and S950. In general, when the number of packets of the MTI filter is larger (that is, when the observation time is longer), a blood flow at a low flow velocity can be observed. However, when the image position is shifted due to an influence of body motion or the like between frames in the packet, performance of the MTI filter deteriorates. Therefore, as illustrated in Fig. 12, desirably, the number of packets of the MTI filter is larger in S950 than that in S920. That is, since there is a high possibility that the reception signal before deformation correction includes an influence of body motion or the like between frames, the number of packets is set to be small in S920. On the other hand, since the influence of body motion or the like can be corrected in the reception signal after deformation correction, the number of packets is set to be large such that a blood flow at a low flow velocity can be observed. Accordingly, the ability to depict a blood flow can be improved.

The super-resolution processing unit 207 extracts a feature point from each of the blood flow images of a plurality of frames, generated in the S950 (S960). A method of determining an extraction range of the feature point may be the same as that of the first embodiment. In addition, the blood flow image from which the feature point is extracted may be either a blood flow image before averaging or after averaging as in the first embodiment. However, since the blood flow image before averaging has a larger pixel value difference in the blood flow, it is easy to extract the feature point.

Finally, the super-resolution processing unit 207 generates a super-resolution blood flow image by integrating pixel values of the feature points respectively extracted from the blood flow images of a large number of frames (S970). The super-resolution processing unit 207 displays the generated super-resolution blood flow image on the display device 108.

In this manner, by setting the MTI filter suitable for correction of the deformation amount and extraction of the blood flow, it is possible to generate a super-resolution blood flow image having improved resolution with high accuracy.

### <Fourth Embodiment>

In a fourth embodiment, as in the third embodiment, blood vessel alignment (deformation correction) is applied not to a blood flow image but to a reception signal, and the MTI filter is applied to the corrected reception signal to generate a blood flow image. Thereafter, as in the second embodiment, a plurality of feature separation images is generated from the blood flow image, and a feature point is extracted from each of the feature separation images. That is, the fourth embodiment is a combination of the second embodiment and the third embodiment.

A device configuration may be the same as that of the first embodiment. A processing flow for acquiring a super-resolution blood flow image according to the fourth embodiment will be described with reference to Fig. 13. The processing from S1110 to S1150 is similar to that from S910 to S950 of the third embodiment. The super-resolution processing unit 207 generates a plurality of feature separation images having different image features from each of the blood flow images of a plurality of frames, generated in S1150 (S1160). Fig. 14 illustrates a frame setting method when a plurality of feature separation images are generated from a blood flow image based on a reception signal subjected to deformation correction. After performing deformation correction on the reception signal, the MTI filter is applied to generate blood flow images, and a plurality of feature separation images are generated from each of the blood flow images. A method of generating the feature separation image may be similar to that of the second embodiment.

The super-resolution processing unit 207 extracts a feature point from each of the plurality of feature separation images generated in S1160 (S1170). A method of determining an extraction range of the feature point may be the same as those in the first and second embodiments.

Finally, the super-resolution processing unit 207 generates a super-resolution blood flow image by integrating pixel values of the feature points extracted from a large number of feature separation images (S1180). The super-resolution processing unit 207 displays the generated super-resolution blood flow image on the display device 108. In the present embodiment as well, similarly to the second embodiment, a frame to be used for integration may be appropriately selected, or a range of image features to be used for integration and a weight at the time of integration may be appropriately set.

In this manner, it is possible to generate a super-resolution blood flow image having improved resolution by setting the MTI filter suitable for correction of a deformation amount and extraction of a blood flow and extracting blood flows having a wide range of flow velocities and thicknesses from blood flow images of a plurality of frames.

### <Other Embodiments>

The above-described embodiments merely illustrate specific examples of the present invention. The scope of the present invention is not limited to the configuration of the above-described embodiment, and various embodiments can be adopted without departing from the gist of the present invention. These embodiments and modifications thereof are included in the scope and gist of the invention and are included in the invention described in the claims and the equivalent scope thereof.

Furthermore, the disclosed technology can take an embodiment as, for example, a system, a device, a method, a program, a recording medium (storage medium), or the like. Specifically, the present invention may be applied to a system including a plurality of devices (for example, a host computer, an interface device, an imaging device, a web application, and the like), or may be applied to a device including one device.

It goes without saying that the object of the present invention is achieved by the following. In other words, a recording medium (or a storage medium) in which a program code (a computer program) of software that implements the functions of the above-described embodiments is recorded is supplied to a system or a device. Such a storage medium is, of course, a computer-readable storage medium. Then, a computer (or a CPU or an MPU) of the system or the device reads and executes the program code stored in the recording medium. In this case, a program code itself read from the recording medium implements the functions of the above-described embodiments, and the recording medium in which the program code is recorded forms the present invention.

The disclosure of the present specification includes the following configurations, a method, and a program.

### (Configuration 1)

An ultrasonic diagnostic device comprising:
a first acquisition unit configured to acquire, based on an ultrasonic signal reflected in a living body, measurement data including tissue-derived information and blood-flow-derived information;
a second acquisition unit configured to acquire, based on blood flow data obtained by extracting or emphasizing the blood-flow-derived information of the measurement data, a deformation amount between a plurality of frames; and
a generation unit configured to generate display image data by aligning feature points in the blood-flow-derived information between the frames based on the deformation amount and then synthesizing the aligned feature points for the plurality of frames.

### (Configuration 2)

The ultrasonic diagnostic device according to configuration 1, wherein
the second acquisition unit is configured to acquire, based on a blood vessel region in the blood flow data, the deformation amount.

### (Configuration 3)

The ultrasonic diagnostic device according to configuration 2, wherein
the second acquisition unit is configured to acquire, based on a position or a shape of a blood vessel region in the blood flow data, the deformation amount.

### (Configuration 4)

The ultrasonic diagnostic device according to configuration 2, wherein
the second acquisition unit is configured to: select, as a reference frame, a frame including the most blood-flow-derived information among the blood flow data of the plurality of frames, and calculate the deformation amount of another frame with reference to the blood vessel region in the blood flow data of the reference frame.

### (Configuration 5)

The ultrasonic diagnostic device according to configuration 1, wherein
the second acquisition unit is configured to acquire the deformation amount by using a learned model trained to output, when the blood flow data is input to the learned model, a deformation amount of the input blood flow data.

### (Configuration 6)

The ultrasonic diagnostic device according to any one of configurations 1 to 5, wherein
the generation unit is configured to: set a blood vessel region as a non-rigid body, and perform alignment between the frames using non-rigid body registration.

### (Configuration 7)

The ultrasonic diagnostic device according to any one of configurations 1 to 6, wherein
the blood flow data is data obtained by reducing a tissue-derived clutter component with respect to the measurement data.

### (Configuration 8)

The ultrasonic diagnostic device according to any one of configurations 1 to 7, wherein
the second acquisition unit is configured to acquire the deformation amount from blood flow data after averaging, the blood flow data being obtained by averaging the blood flow data of two or more frames.

### (Configuration 9)

The ultrasonic diagnostic device according to any one of configurations 1 to 8, wherein
the generation unit is configured to extract, as the feature point, a pixel having a pixel value within a predetermined extraction range from an image of each of the plurality of frames, the image having the blood-flow-derived information as the pixel value.

### (Configuration 10)

The ultrasonic diagnostic device according to configuration 9, wherein
the generation unit is configured to set the extraction range according to a maximum pixel value of the image.

### (Configuration 11)

The ultrasonic diagnostic device according to configuration 9 or 10, wherein
the generation unit is configured to: divide the image into a plurality of local regions, and set, for each of the local regions, the extraction range according to a maximum pixel value of each of the local regions.

### (Configuration 12)

The ultrasonic diagnostic device according to any one of configurations 9 to 11, wherein
the generation unit is configured to: divide the image into a plurality of local regions, and set, for each of the local regions, the extraction range according to a depth from a body surface of each of the local regions.

### (Configuration 13)

The ultrasonic diagnostic device according to any one of configurations 9 to 12, wherein
the generation unit is configured to generate the display image data by removing noise from pixel groups extracted as the feature points from the images of the plurality of frames and synthesizing the pixel groups obtained by removing the noise therefrom.

### (Configuration 14)

The ultrasonic diagnostic device according to any one of configurations 9 to 13, wherein
the generation unit is configured to: up-convert the images of the plurality of frames, and extract the feature points from the up-converted images.

### (Configuration 15)

The ultrasonic diagnostic device according to any one of configurations 9 to 14, wherein
the generation unit is configured to: perform, on the images of the plurality of frames, correction for alignment based on the deformation amount, and extract the feature points from the respective corrected images of the plurality of frames.

### (Configuration 16)

The ultrasonic diagnostic device according to any one of configurations 9 to 14, wherein
the generation unit is configured to: perform, on the measurement data of the plurality of frames, correction for alignment based on the deformation amount, generate, based on the corrected measurement data of the plurality of frames, images of the plurality of frames, each of the images having the blood-flow-derived information as the pixel value, and extract the feature points from the generated respective images of the plurality of frames.

### (Configuration 17)

The ultrasonic diagnostic device according to configuration 15 or 16, wherein
the generation unit is configured to: generate, from the respective images of the plurality of frames, a plurality of feature separation images having image features different from each other, and extract the feature points from respective ones of the plurality of feature separation images.

### (Configuration 18)

The ultrasonic diagnostic device according to configuration 17, wherein
each of the image features includes at least one of an image feature related to a flow velocity of a blood flow, an image feature related to a shape or a thickness of a blood vessel, and an image feature related to a direction of the blood flow.

### (Configuration 19)

The ultrasonic diagnostic device according to configuration 17 or 18, wherein
the generation unit is configured to synthesize, when generating the display image data, the feature points extracted from the respective ones of the plurality of feature separation images by weighting the feature points according to the image features.

### (Configuration 20)

A medical information processing device comprising a generation unit configured to:
acquire, based on blood flow data obtained by extracting or emphasizing blood-flow-derived information of measurement data including tissue-derived information and the blood-flow-derived information in a living body, a deformation amount between a plurality of frames, and
generate display image data by aligning feature points in the blood-flow-derived information between the frames based on the deformation amount and then synthesizing the aligned feature points for the plurality of frames.

### (Method 21)

An information processing method of a computer, comprising:
acquiring, based on an ultrasonic signal reflected in a living body, measurement data including tissue-derived information and blood-flow-derived information;
acquiring, based on blood flow data obtained by extracting or emphasizing the blood-flow-derived information of the measurement data, a deformation amount between a plurality of frames; and
generating display image data by aligning feature points in the blood-flow-derived information between the frames based on the deformation amount and then synthesizing the aligned feature points for the plurality of frames.

### (Program 22)

A program that causes a computer to execute an information processing method comprising:
acquiring, based on an ultrasonic signal reflected in a living body, measurement data including tissue-derived information and blood-flow-derived information;
acquiring, based on blood flow data obtained by extracting or emphasizing the blood-flow-derived information of the measurement data, a deformation amount between a plurality of frames; and
generating display image data by aligning feature points in the blood-flow-derived information between the frames based on the deformation amount and then synthesizing the aligned feature points for the plurality of frames.

The present invention is not limited to the embodiment described above, and various modifications and amendments may be implemented thereon without departing from the spirit and scope of the present invention. Accordingly, the following claims are attached in order to publicize the scope of the present invention.

The present application claims priority on the basis of Japanese Patent Application No. 2023-100608, filed on June 20, 2023, and all of the contents disclosed therein are included by reference.

### [Reference Signs List]

1: Ultrasonic diagnostic device

## Claims

1. An ultrasonic diagnostic device comprising:
a first acquisition unit configured to acquire, based on an ultrasonic signal reflected in a living body, measurement data including tissue-derived information and blood-flow-derived information;
a second acquisition unit configured to acquire, based on blood flow data obtained by extracting or emphasizing the blood-flow-derived information of the measurement data, a deformation amount between a plurality of frames; and
a generation unit configured to generate display image data by aligning feature points in the blood-flow-derived information between the frames based on the deformation amount and then synthesizing the aligned feature points for the plurality of frames.

2. The ultrasonic diagnostic device according to claim 1, wherein
the second acquisition unit is configured to acquire, based on a blood vessel region in the blood flow data, the deformation amount.

3. The ultrasonic diagnostic device according to claim 2, wherein
the second acquisition unit is configured to acquire, based on a position or a shape of a blood vessel region in the blood flow data, the deformation amount.

4. The ultrasonic diagnostic device according to claim 2, wherein
the second acquisition unit is configured to: select, as a reference frame, a frame including the most blood-flow-derived information among the blood flow data of the plurality of frames, and calculate the deformation amount of another frame with reference to the blood vessel region in the blood flow data of the reference frame.

5. The ultrasonic diagnostic device according to claim 1, wherein
the second acquisition unit is configured to acquire the deformation amount by using a learned model trained to output, when the blood flow data is input to the learned model, a deformation amount of the input blood flow data.

6. The ultrasonic diagnostic device according to claim 1, wherein
the generation unit is configured to: set a blood vessel region as a non-rigid body, and perform alignment between the frames using non-rigid body registration.

7. The ultrasonic diagnostic device according to claim 1, wherein
the blood flow data is data obtained by reducing a tissue-derived clutter component with respect to the measurement data.

8. The ultrasonic diagnostic device according to claim 1, wherein
the second acquisition unit is configured to acquire the deformation amount from blood flow data after averaging, the blood flow data being obtained by averaging the blood flow data of two or more frames.

9. The ultrasonic diagnostic device according to claim 1, wherein
the generation unit is configured to extract, as the feature point, a pixel having a pixel value within a predetermined extraction range from an image of each of the plurality of frames, the image having the blood-flow-derived information as the pixel value.

10. The ultrasonic diagnostic device according to claim 9, wherein
the generation unit is configured to set the extraction range according to a maximum pixel value of the image.

11. The ultrasonic diagnostic device according to claim 9, wherein
the generation unit is configured to: divide the image into a plurality of local regions, and set, for each of the local regions, the extraction range according to a maximum pixel value of each of the local regions.

12. The ultrasonic diagnostic device according to claim 9, wherein
the generation unit is configured to: divide the image into a plurality of local regions, and set, for each of the local regions, the extraction range according to a depth from a body surface of each of the local regions.

13. The ultrasonic diagnostic device according to claim 9, wherein
the generation unit is configured to generate the display image data by removing noise from pixel groups extracted as the feature points from the images of the plurality of frames and synthesizing the pixel groups obtained by removing the noise therefrom.

14. The ultrasonic diagnostic device according to claim 9, wherein
the generation unit is configured to: up-convert the images of the plurality of frames, and extract the feature points from the up-converted images.

15. The ultrasonic diagnostic device according to claim 9, wherein
the generation unit is configured to: perform, on the images of the plurality of frames, correction for alignment based on the deformation amount, and extract the feature points from the respective corrected images of the plurality of frames.

16. The ultrasonic diagnostic device according to claim 9, wherein
the generation unit is configured to: perform, on the measurement data of the plurality of frames, correction for alignment based on the deformation amount, generate, based on the corrected measurement data of the plurality of frames, images of the plurality of frames, each of the images having the blood-flow-derived information as the pixel value, and extract the feature points from the generated respective images of the plurality of frames.

17. The ultrasonic diagnostic device according to claim 15 or 16, wherein
the generation unit is configured to: generate, from the respective images of the plurality of frames, a plurality of feature separation images having image features different from each other, and extract the feature points from respective ones of the plurality of feature separation images.

18. The ultrasonic diagnostic device according to claim 17, wherein
each of the image features includes at least one of an image feature related to a flow velocity of a blood flow, an image feature related to a shape or a thickness of a blood vessel, and an image feature related to a direction of the blood flow.

19. The ultrasonic diagnostic device according to claim 17, wherein
the generation unit is configured to synthesize, when generating the display image data, the feature points extracted from the respective ones of the plurality of feature separation images by weighting the feature points according to the image features.

20. A medical information processing device comprising a generation unit configured to:
acquire, based on blood flow data obtained by extracting or emphasizing blood-flow-derived information of measurement data including tissue-derived information and the blood-flow-derived information in a living body, a deformation amount between a plurality of frames, and
generate display image data by aligning feature points in the blood-flow-derived information between the frames based on the deformation amount and then synthesizing the aligned feature points for the plurality of frames.

21. An information processing method of a computer, comprising:
acquiring, based on an ultrasonic signal reflected in a living body, measurement data including tissue-derived information and blood-flow-derived information;
acquiring, based on blood flow data obtained by extracting or emphasizing the blood-flow-derived information of the measurement data, a deformation amount between a plurality of frames; and
generating display image data by aligning feature points in the blood-flow-derived information between the frames based on the deformation amount and then synthesizing the aligned feature points for the plurality of frames.

22. A program that causes a computer to execute an information processing method comprising:
acquiring, based on an ultrasonic signal reflected in a living body, measurement data including tissue-derived information and blood-flow-derived information;
acquiring, based on blood flow data obtained by extracting or emphasizing the blood-flow-derived information of the measurement data, a deformation amount between a plurality of frames; and
generating display image data by aligning feature points in the blood-flow-derived information between the frames based on the deformation amount and then synthesizing the aligned feature points for the plurality of frames.
